# EUROPEAN PATENT SPECIFICATION

(11) **EP 2 247 270 B1**
(45) Date of publication and mention of the grant of the patent: **26.03.2014**
(21) Application number: 09708601.1
(22) Date of filing: 04.02.2009
(51) Int. Cl.: A61F 9/007, A61M 5/32

(54) **NEEDLE FOR OPHTHALMIC PROCEDURES**
NADEL FÜR OPHTHALMISCHE VERFAHREN
AIGUILLE POUR DES INTERVENTIONS OPHTALMIQUES

(30) Priority: 05.02.2008 GB 0802044
(43) Date of publication of application: 10.11.2010
(73) Proprietor: Helica Instruments Limited, Edinburgh EH14 4AP (GB)
(72) Inventor: HOWIESON, Maurice Maxwell, West Lothian EH52 5RF (GB)
(74) Representative: Fulton, David James
(86) International application number: PCT/GB2009/050104
(87) International publication number: WO 2009/098508

(56) References cited:
- EP-A- 1 872 819
- WO-A-01/28473
- DE-U1-202007 009 977
- US-A1- 2001 037 092

## Description

The present invention relates to medical apparatus and in particular to an atraumatic, flexible sub-Tenon needle for performing ophthalmic surgical procedures.

An increasing number of ophthalmic surgical procedures are performed under local anaesthesia. Up until recently both peribulbar and retrobulbar blocks were the techniques of choice, and in experienced hands often produce excellent operating conditions in terms of both analgesia and akinesia. These techniques have, however, been associated with potentially serious complications, including retrobulbar haemorrhage, globe perforation, optic nerve damage and inadvertent brainstem anaesthesia.

More recently the alternative technique of sub-Tenon's anaesthesia has been adopted so as to overcome the complications associated with the prior art ophthalmic procedures. In order to perform this technique a blunt, curved, stainless steel needle (known as a Stevens needle) is employed to administer anaesthetic into the sub-Tenon's space of the eye.

Although, an improvement on both peribulbar and retrobulbar block techniques, there are still a number of problematic features associated with the known apparatus employed to perform sub-Tenon's anaesthesia techniques. In the first instance, the rigid nature of the Stevens needle requires a significant degree of skill and manual dexterity, on behalf of the surgeon deploying the apparatus, so as not to rupture the Tenon's capsule. Furthermore, to perform sub-Tenon's anaesthesia technique with a Stevens needle requires a number of incisions to be made in the conjunctiva and the Tenon's capsule so as to administer sufficient aesthetic into the sub-Tenon's space. Typically, this involves making two separate incisions, one either side of the patient's sclera.

If a Stevens needle is not available to a surgeon it is known for them to alternatively employ straight blunt needles or the straight plastic portion of an intravenous cannula to perform the technique. Locating the sub-Tenon's space without inadvertently damaging a patient's eye is however significantly more difficult when straight needles or cannulas are employed. In addition, the diameter of an intravenous cannula requires larger incisions to be made when compared with a Stevens needle, thus further increasing the risk to, and recovery times for, patients.

A prior art needle suitable for performing ophthalmic surgical procedures is disclosed by WO 01/28473 A. The preamble of claim 1 is based on this document.

It is therefore an object of the present invention to provide a needle for performing ophthalmic surgical procedures that obviates, or at least mitigates, one or more of the above described problems experienced by the needles known in the art.

### Summary of Invention

According to the present invention there is provided a needle suitable for performing ophthalmic surgical procedures, wherein the needle comprises an adapter that provides an attachment means for the needle to a syringe and a flexible curved tube attached to a distal end of the adapter.

Employing a needle with a flexible curved tube significantly reduces the risk of trauma of a patient's eye during an ophthalmic surgical procedure e.g. a sub-Tenon's block procedure.

The needle may further comprise a connector adapted to attach the flexible curved tube to the distal end of the adapter.

The flexible curved tube may comprise a plastic material. Preferably the plastic material comprises polytetrafluoroethylene (PTFE).

Preferably the flexible curved tube has an outer diameter of between 0.5 and 1.25mm. In one embodiment the flexible curved tube may have an outer diameter of 1 mm.

Preferably the flexible curved tube has an inner diameter of between 0.25 and 1mm. In one embodiment the flexible curved tube may have an inner diameter of 0.5mm.

Typically the flexible curved tube has a length between 20mm and 40mm. In one embodiment the flexible curved tube has a length of 30mm.

The flexible curved tube may have a distal end that comprises a profile selected from the group comprising chisel, oval, rounded and tapered end profiles.

The adapter may comprise an open ended tapered tube.

Typically the connector comprises a rigid cannula around which is located a collar. The cannula may be of stainless steel or other suitable rigid material, and the collar may be of plastics material.

Typically the outer surface of the collar provides an interference fit with an inner surface of the open ended tapered tube of the adapter.

Typically the outer surface of the rigid cannula provides an interference fit with an inner surface of the flexible curved tube.

Optionally the needle further comprises a protective sleeve, the inner surface of which provides an interference fit with the outer surface of the open ended tapered tube of the adapter.

According to the present invention there is also provided a method of delivering an anaesthetic to an eye having a Tenon's casule and sub-Tenon's space in an ophthalmic surgical procedure comprising the steps of:
(a) providing a syringe containing an anaesthetic, the syringe being connected to a needle by an adaptor, the needle comprising a flexible plastic tube;
(b) forming an incision in a patient's eye to breach the Tenon's capsule of the eye;
(c) inserting a flexible plastic tube of the needle through the incision to follow a curvature of a globe of the eye;
(d) injecting the anaesthetic into the sub-Tenon's space of the eye.

There is also provided a method of delivering a flushing agent to a tear duct of an eye in an ophthalmic surgical procedure comprising the steps of:
(a) providing a syringe containing a flushing agent, the syringe being connected to a needle by an adaptor, the needle comprising a flexible plastic tube;
(b) inserting a flexible plastic tube of the needle through the tear duct to follow a curvature of a globe of the eye; and
(c) injecting the flushing agent through the flexible plastic tube of the needle into the tear duct of the eye.

The flushing agent may be a solution, particularly a saline solution.

There is also provided a method of performing an ophthalmic surgical procedure comprising the steps of:
(a) providing a needle, said needle comprising an adapter that provides an attachment means for the needle to a syringe and a flexible curved tube attached to a distal end of the adapter;
(b) making an incision; and
(c) inserting the flexible curved tube trough the incision.

### Brief Description of Drawings

Aspects of the present invention will now be described by way of example only and with reference to the accompanying figures, in which:
Figure 1 shows a schematic diagram of a human eye with a distal end of a needle, in accordance with an aspect of the present invention, located within the sub-Tenon's space;
Figure 2 shows:
   (a) a picture of the needle of Figure 1;
   (b) a picture of an exploded representation of the needle of Figure 1; and
   (c) a picture of the needle of Figure 1 housed within a protective sleeve; and
Figure 3 presents four alternatively shaped ends of the flexible curved tube.

For consistency and clarity purpose the various features of the described needle are referred to by the same reference numerals throughout the specification.

### Detailed Description

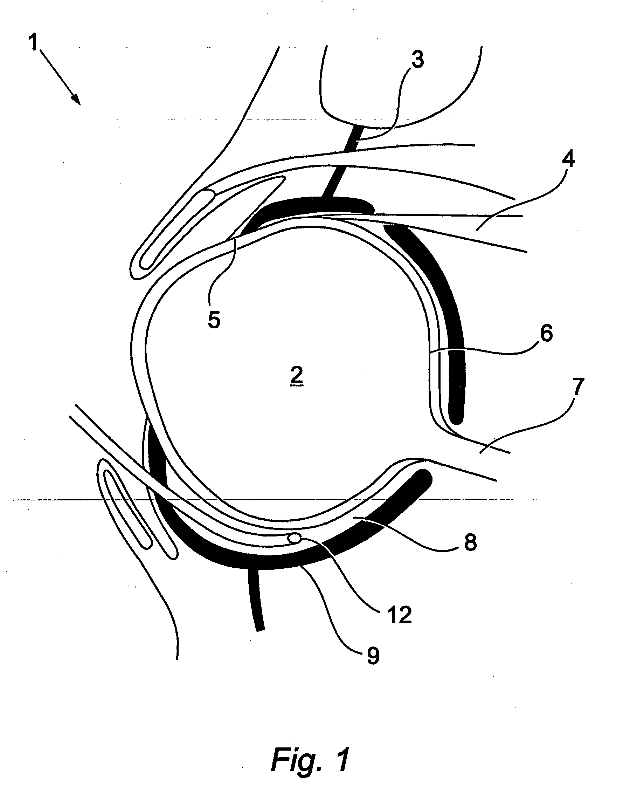

Aspects and embodiments of the present invention will now be described with reference to Figures 1 to 3.

For ease of reference, Figure 1 presents a schematic diagram of a human eye. Various component of the eye 1 can be seen. In particular a globe, orbital septum, superior rectus, conjunctiva, sclera, optic nerve, sub-Tenon's space and Tenon's capsule of the eye are identified within Figure 1.

The Tenon's capsule 9 is a thin layer of connective tissue which surrounds the globe 2. Anteriorly it lies in close apposition to the conjunctiva 5 and fuses with it at the level of the limbus. It extends posteriorly in all directions around the globe 2, ultimately fusing with the dura of the optic nerve 7. At intervals along its course it is pieced by the extra-ocular muscles as they insert into the globe 2. The sub-Tenon's space 8 is a potential space between the Tenon's capsule 9 and the sclera 6.

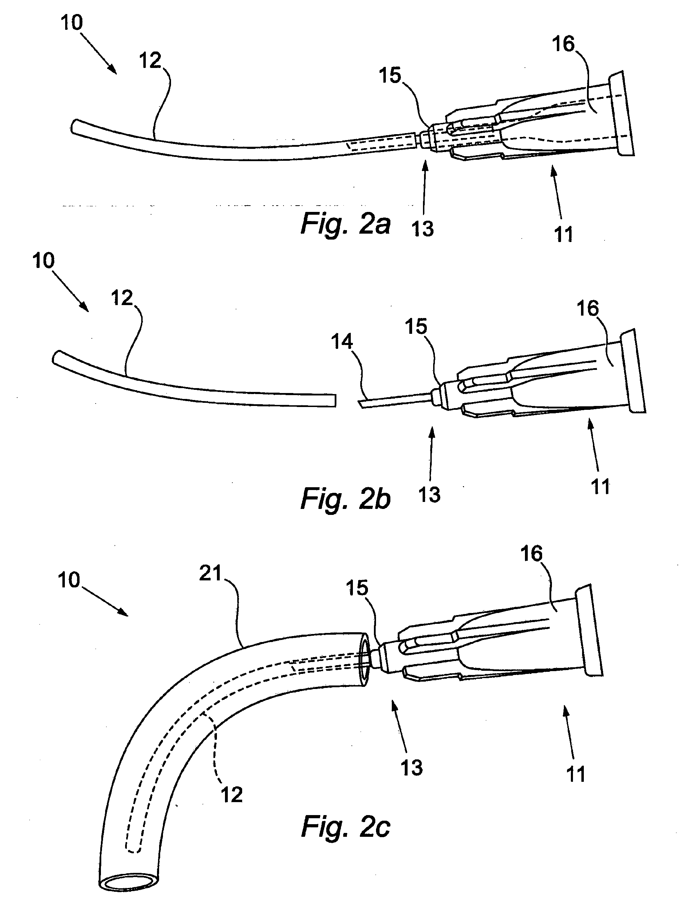

Figure 2 presents three representations of a needle in accordance with the present invention. In particular, Figures 2(a) and (b) present an unexploded and an exploded picture, respectively, of the needle 10. The needle 10 can be seen to comprise three main components, namely an adapter 11, a flexible plastic tube 12 and a connector 13 employed to attach the plastic tube 12 to the adapter 11. Further detail of each of these elements will now be described.

The connector 13 comprises a rigid metal cannula around which is located a plastic collar. From Figure 2 it can be seen that the adapter 11 essentially comprises an open ended, tapered, plastic tube. The opening of widest diameter of the tapered plastic tube 16 is sized so as to allow the shaped inner surface of the plastic tube 16 to mechanically connect to a syringe (not shown) with an interference fit or similar. In a similar manner the opening of smaller diameter of the tapered plastic tube 16 is sized such that the inner surface of the tapered plastic tube 16 forms an interference fit with the outer surface of the plastic collar 15 of the connector 13. When the tapered plastic tube 16 and the plastic collar 15 are so connected a portion of the metal cannula 14 protrudes from the adapter 11. It is the protruding end of the metal cannula 14 that provides a connection means for the flexible plastic tube 12. This is achieved by way of an interference fit between the inner surface of the flexible plastic tube 12 and the outer surface of the metal cannula 14.

The flexible plastic tube 12 comprises polytetrafluoroethylene (PTFE) having an outer diameter of about 1 mm, an inner diameter of about 0.5mm and a length of approximately 30mm. The length and curvature of the plastic tube 12 is chosen such that it is commensurate with the outer surface of the sclera 6, as shown in Figure 1. Given the flexible nature of PTFE the radius of curvature is easily adjusted so as to be suitable for use with either the eye of an adult or a child. The flexible plastic tube is sufficiently flexible to allow some deformation when subject to external force, but rigid enough to remain in its curved form when not subject to external force. It is to be understood that the invention is not limited to PTFE and other materials may be used for the flexible curved tube, provided that they exhibit appropriate mechanical properties and may be sterilised.

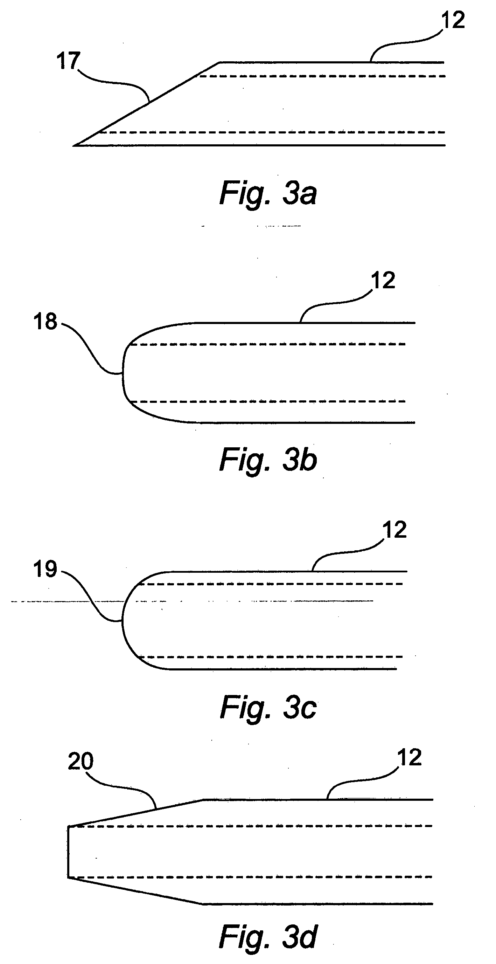

Figure 3 presents four different end profiles for the plastic tube 12 namely: (a) chisel, (b) oval, (c) rounded and tapered. These end profiles assist with the insertion of the plastic tube 12 through an incision into Sub-Tenon's space 8.

It will be appreciated by those skilled in the art that the above defined dimensions of the flexible plastic tube 12 are given by way of example only. The outer diameter of the flexible plastic tube 12 may vary between 0.5 and 1.25mm, while the inner diameter may vary between 0.25 and 1 mm, depending on the dimensions of the corresponding outer diameter. The length of the flexible plastic tube 12 may also vary between 20mm and 40mm.

From Figure 2(c) it can be seen that the needle 10 may be provided with a protective sleeve. The protective sleeve 21 has a curved profile such that it locates over the flexible plastic tube 12 and thus acting to maintain the shape of the flexible plastic tube 12 during storage. The protective sleeve 21 is sized so as to provide an interference fit with the outer surface of the plastic tube 16 of the adapter 11, so that it remains connected to the adapter 11 until manually removed.

The use of a needle according to the invention in performing the sub-Tenon's block is now described.

In the first instance a patient's suitability for a local anaesthetic technique is required to be established. Factors that require to be considered include the age and general health of the patient. It is also important that the patient should be capable lying still for the duration of surgery.

Once it is established that a patient is suitable for such a technique they are then placed in the supine position and standard monitoring applied (ECG, pulse oximetry and non-invasive blood pressure). The next step is for the conjunctiva 5 to be anaesthetised with a topical anaesthetic solution such as proxymetacaine and amethocaine. The anesthetised conjunctiva 5 is then cleaned by carefully placing a few drops of povidone iodine beneath the lower eyelid. The rest of the orbital margin can then be cleaned with any remaining solution.

An eyelid speculum (not shown) is then inserted to improve access and prevent blinking of the patient's eye 1. A small tent of conjunctiva (together with the underlying Tenon's capsule) is raised with a pair of blunt, non-toothed forceps approximately 5-10 mm from the infero-nasal limbus. A small incision is made in the tissue using a pair of ophthalmic scissors, exposing the white sclera 6. A failure to visualize the sclera 6 often indicates that the Tenon's capsule 9 has not been breached. In this circumstance a second attempt should be made at picking up the Tenon's capsule 9 and a further incision made.

With the needle 10 attached to a syringe containing a local anaesthetic the protective sleeve 21 is then removed so as to expose the flexible plastic tube 12. The plastic tube 12 is then inserted and passed posteriorly such that it follows the curvature of the globe 2, until its tip is perceived to have passed the equator, see Figure 1. The local anaesthetic is then injected into the sub-Tenon's space 8.

Numerous local anesthetic solutions can be used with varying degrees of efficacy and duration. For example in the UK, most anaesthetists use a combination of lidocaine (2%) and bupivacaine (0.5%) often in equal proportion, with or without the addition of hyaluronidase (30 - 150 iu/ml).

A local anaesthetic mixture volume of 3-5 ml is generally sufficient to produce a good quality block, with a duration of surgical anaesthesia exceeding one hour.

For surgery of short duration, such as cataract extraction, lidocaine alone may be used. The addition of hyaluronidase is not essential, but is well recognized to increase the spread of local anaesthetic in the retro-orbital compartment. It is, however, expensive and has been associated with severe side effects such as anaphylaxis and sterile abscess formation.

An alternative to the lidocaine-bupivacaine mixture is articaine (2%) which has been widely used in dental anaesthesia. Articaine has the advantage of increased tissue perfusion, thus obviating the need for hyaluronidase. It also has a low systemic toxicity

On injection of the local anaesthetic, little resistance should be encountered and most of the solution should disappear behind the globe 2 resulting in slight proptosis. If resistance is encountered, the needle 10 can be withdrawn slightly and repositioned. On occasions, there may be a moderate amount of leakage back onto the surface of the eye and mild chemosis is not uncommon. After removal of the tube 12, gentle pressure applied to the globe 2 for about one minute may assist in the spread of the local anaesthetic.

The onset of analgesia is usually rapid, whereas maximal akinesia may take up to 10 minutes to develop. Should the block not appear to be adequate after 5 minutes, as indicated by the absence of a developing akinesia, a further top up can be given through the pre-existing incision. The flexible nature of the plastic tube 12 allows for manipulation of the tip, via the single incision, such that it can be located within the sub-Tenon's space 8 on the opposite side of the globe 2 from which the incision is made. In this way the top up can be applied to a separate area of the eye 1 to that of the original administration of the local anaesthetic, thus increasing the likelihood of the occurrence of akinesia.

The employment of the needle of the present invention shares many of the advantages of the known apparatus for performing a sub-Tenon's block. In addition, the likelihood of success of the sub-Tenon's block carried out with the needle of the present invention is increased when compared to those known procedures carried out with either straight needles or with a Stevens needle. Complications are rare since the risk of trauma during the procedure is significantly reduced. This is a direct result of the flexible nature of the plastic tube 12, which reduces the opportunity for accidentally rupturing the Tenon's capsule while reducing the requirement for more than one incision to be made on a patient's eye. The shaped end profile also assists in reducing the opportunity for rupture and reducing the requirement for multiple incisions.

The use of a needle to deliver anaesthetic to a tear duct is now described.

The needle of the invention can be used to deliver a flushing agent or solution to a tear duct, in order to clear a blocked tear duct. In such circumstances a smaller diameter needle may be used, since the needle passes through the tear duct without the need for an incision. However the advantages of a flexible curved tube 12 which corresponds in curvature to the globe 2 of the eye remain. Typically the flushing agent is a saline solution. In one example the flexible plastic tube 12 comprises polytetrafluoroethylene (PTFE) and has an outer diameter of about 0.4 to 0.75mm, preferably about 0.5 mm, an inner diameter of about 0.25 to 0.45 mm, preferably about 0.3 mm, and a length of about 20 to 30 mm, preferably approximately 25 mm.

The foregoing description of the invention has been presented for the purpose of illustration and description, and is not intended to be exhaustive or to limit the invention to the precise form disclosed. The described embodiments were chosen and described in order to best explain the principles of the invention and its practical applications, to thereby enable others skilled in the art to best utilise the invention in various embodiments and with various modifications as are suited to the particular use upon completion. Therefore, further modifications and improvements may be incorporated without departing from the scope of the invention herein intended.

## Claims

1. A needle (10) suitable for performing ophthalmic surgical procedures, wherein the needle (10) comprises an adapter (11) that provides an attachment means for the needle (10) to a syringe
**characterised in that**
the needle further comprises a flexible curved tube (12) attached to a distal end of the adapter (11).

2. A needle (10) according to claim 1 further comprising a connector (13) adapted to attach the flexible curved tube (12) to the distal end of the adapter (11).

3. A needle (10) according to claim 1 or 2 wherein the flexible curved tube (12) comprises a plastic material.

4. A needle (10) according to claim 1 or 2 wherein the flexible curved tube (12) comprises polytetrafluoroethylene (PTFE).

5. A needle (10) according to any preceding claim wherein the flexible curved tube (12) has an outer diameter of between 0.4 and 1.25mm.

6. A needle (10) according to any preceding claim wherein the flexible curved tube (12) has an inner diameter of between 0.25 and 1 mm.

7. A needle (10) according to any preceding claim wherein the flexible curved tube (12) has a length of between 20mm and 40mm.

8. A needle (10) according to any preceding claim wherein the flexible curved tube (12) has a distal end that comprises a profile selected from the group comprising chisel (17), oval (18), rounded (19) and tapered (20) end profiles.

9. A needle (10) according to any preceding claim wherein the adapter (11) comprises an open ended tapered tube (16).

10. A needle (10) according to any of claims 2 to 9 wherein the connector (13) comprises a rigid cannula (14) around which is located a collar (15).

11. A needle (10) according to claim 10 when dependent on claim 9 wherein the collar (15) has an outer surface which provides an interference fit with an inner surface of the adapter (11).

12. A needle (10) according to claim 10 or 11 wherein the rigid cannula (14) has an outer surface which provides an interference fit with an inner surface of the flexible curved tube (12).

13. A needle (10) according to any of the preceding claims wherein the needle (10) further comprising a protective sleeve having an inner surface which provides an interference fit with an outer surface of the adapter (11).

## Patentansprüche

1. Eine Nadel (10) zur Durchführung von Augenoperationen, wobei die Nadel (10) einen Adapter (11) beinhaltet, über den die Nadel (10) an einer Spritze befestigt werden kann,
**dadurch gekennzeichnet, dass**
die Nadel außerdem einen gebogenen Schlauch (12) beinhaltet, der am Distalende des Adapters (11) befestigt ist.

2. Eine Nadel (10) nach Anspruch 1, die außerdem ein geeignetes Anschlussstück (13) besitzt, über das der gebogene Schlauch (12) mit dem Distalende des Adapters (11) verbunden werden kann.

3. Eine Nadel (10) nach Anspruch 1 oder 2, wobei der gebogene Schlauch (12) Kunststoffmaterial enthält.

4. Eine Nadel (10) nach Anspruch 1 oder 2, wobei der gebogene Schlauch (12) Polytetrafluorethylen (PTFE) enthält.

5. Eine Nadel (10) nach einem der vorangehenden Ansprüche, wobei der gebogene Schlauch (12) einen Außendurchmesser zwischen 0,4 und 1,25 mm besitzt.

6. Eine Nadel (10) nach einem der vorangehenden Ansprüche, wobei der gebogene Schlauch (12) einen Innendurchmesser zwischen 0,25 und 1 mm besitzt.

7. Eine Nadel (10) nach einem der vorangehenden Ansprüche, wobei der gebogene Schlauch (12) eine Länge zwischen 20 und 40 mm besitzt.

8. Eine Nadel (10) nach einem der vorangehenden Ansprüche, wobei der gebogene Schlauch (12) ein Distalende mit einem Profil besitzt, das aus einer Gruppe mit meißelförmigen (17), ovalen (18), gerundeten (19) und konischen (20) Endprofilen ausgewählt wurde.

9. Eine Nadel (10) nach einem der vorangehenden Ansprüche, wobei der Adapter (11) ein konisches Röhrchen mit einem offenen Ende besitzt.

10. Eine Nadel (10) nach einem der Ansprüche 2 bis 9, wobei das Anschlussstück (13) eine starre Kanüle (14) mit einem umliegenden Halsstück (15) besitzt.

11. Eine Nadel (10) nach Anspruch 10, soweit rückbezogen auf Anspruch 9, wobei das Halsstück (15) eine Außenfläche besitzt, die für die Presspassung mit einer Innenfläche des Adapters (11) sorgt.

12. Eine Nadel (10) nach Anspruch 10 oder 11, wobei die starre Kanüle (14) eine Außenfläche besitzt, die für die Presspassung mit einer Innenfläche des gebogenen Schlauchs (12) sorgt.

13. Eine Nadel (10) nach einem der vorangehenden Ansprüche, wobei die Nadel (10) außerdem eine Schutzhülse mit einer Innenfläche besitzt, die für die Presspassung mit einer Außenfläche des Adapters (11) sorgt.

## Revendications

1. Une aiguille (10) permettant d'effectuer les procédures chirurgicales ophtalmologiques, l'aiguille (10) comportant un adaptateur (11) avec une attache pour fixer l'aiguille (10) sur une seringue, et l'aiguille comportant un tube incurvé souple (12) fixé à l'extrémité distale de l'adaptateur (11).

2. Une aiguille (10) conforme à la revendication 1 comprenant en outre un raccord (13) permettant de raccorder le tube incurvé souple (12) à l'extrémité distale de l'adaptateur (11).

3. Une aiguille (10) conforme à la revendication 1 ou 2, le tube incurvé souple (12) étant en matière plastique.

4. Une aiguille (10) conforme à la revendication 1 ou 2, le tube incurvé souple (12) étant en polytétrafluoroéthylène (PTFE).

5. Une aiguille (10) conforme à une revendication précédente, le tube incurvé souple (12) ayant un diamètre extérieur entre 0,4 et 1,25 mm.

6. Une aiguille (10) conforme à une revendication précédente, le tube incurvé souple (12) ayant un diamètre intérieur entre 0,25 et 1 mm.

7. Une aiguille (10) conforme une revendication précédente, le tube incurvé souple (12) ayant une longueur entre 20 et 40 mm.

8. Une aiguille (10) conforme à une revendication précédente, le tube incurvé souple (12) ayant une extrémité distale dont le profil peut être sélectionné parmi les profils suivants : ciseau (17), oval (18), arrondi (19) et conique (20).

9. Une aiguille (10) conforme à une revendication précédente, l'adaptateur (11) comportant un tube à embout conique ouvert (16).

10. Une aiguille (10) conforme à l'une des revendications 2 à 9, le raccord (13) comportant une canule rigide (14) autour de laquelle est placé un collier (15).

11. Une aiguille (10) conforme à la revendication 10 dans laquelle, lorsque la revendication 9 s'applique, le collier (15) a une surface extérieure permettant de l'emmancher dans la surface intérieure de l'adaptateur (11).

12. Une aiguille (10) conforme à la revendication 10 ou 11 dans laquelle la canule rigide (14) comporte une surface extérieure permettant de l'emmancher dans la surface intérieure du tube incurvé souple (12).

13. Une aiguille (10) conforme à l'une des revendications précédentes dans laquelle l'aiguille (10) comporte en outre un manchon protecteur dont la surface intérieure permet de l'emmancher à la surface extérieure de l'adaptateur (11).
